# FASCICULE DE BREVET EUROPEEN

(11) **EP 1 335 775 B1**
(45) Date de publication et mention de la délivrance du brevet: **17.05.2006**
(21) Numéro de dépôt: 01997329.6
(22) Date de dépôt: 23.11.2001
(51) Int. Cl.: A61N 1/378, A61N 1/372, A61F 5/00, A61F 2/02

(54) **DISPOSITIF DE MISE EN OEUVRE A DISTANCE ET SANS LIEN MATERIEL D'UN IMPLANT ET IMPLANT MIS EN OEUVRE PAR CE DISPOSITIF**
VORRICHTUNG ZUM DRAHTLOSEN FERNBETREIBEN EINES IMPLANTATS SOWIE IMPLANTAT HIERZU
DEVICE FOR REMOTE AND NON-CONNECTED IMPLEMENTATION OF AN IMPLANT AND IMPLANT IMPLEMENTED BY SAID DEVICE

(30) Priorité: 23.11.2000 FR 0015140
(43) Date de publication de la demande: 20.08.2003
(73) Titulaire: Cancel, Richard, 83130 La Garde (FR); Wallace, Richard, 83590 Gonfaron (FR); Sassi, Gérard, 83200 Toulon (FR)
(72) Inventeur: Cancel, Richard, 83130 La Garde (FR); Wallace, Richard, 83590 Gonfaron (FR); Sassi, Gérard, 83200 Toulon (FR)
(74) Mandataire: Breese, Pierre
(86) Numéro de dépôt international: PCT/FR2001/003704
(87) Numéro de publication internationale: WO 2002/041945

(56) Documents cités:
- EP-A- 0 619 101
- WO-A-00/15158
- WO-A-00/30534
- WO-A-96/22049
- WO-A-98/43700
- US-A- 5 354 319

## Description

La présente invention se rapporte au domaine de la mise en oeuvre à distance et sans lien matériel d'implants comportant des appareillages électriques.

La présente invention se rapporte plus particulièrement à un dispositif de commande à distance et sans lien matériel d'un dispositif de constriction gastrique comportant un appareillage électrique, ledit dispositif de commande comportant une source d'induction constituant une source d'énergie pour le fonctionnement dudit appareillage électrique.

L'art antérieur connaît déjà des dispositifs de mise en oeuvre à distance et sans lien matériel d'implants comportant des appareillages électriques et notamment la demande internationale de brevet WO 00/15158.

Cette demande de brevet porte sur un dispositif de constriction gastrique d'un type particulier qui peut être mis en oeuvre à distance, sans lien matériel, grâce à un émetteur comportant une source d'induction. La source d'induction transmet l'énergie nécessaire pour la mise en oeuvre du moteur et des fréquences particulières sont sélectionnées, l'une pour provoquer un ordre de marche avant ; l'autre pour provoquer un ordre de marche arrière.

L'inconvénient majeur de ce dispositif réside dans le fait que les seuls ordres transmissibles sont : marche avant/marche arrière. Il n'y a aucun moyen de vérifier, par l'intermédiaire du dispositif, que l'ordre a été reçu, qu'il a été exécuté et dans quelle mesure il l'a été. En outre, ce dispositif ne donne pas toutes les garanties nécessaires contre une mise en oeuvre intempestive de l'appareillage électrique de l'implant.

Il existe, par ailleurs des dispositifs de mise en oeuvre susceptibles de communiquer avec des implants, mais dans ces systèmes, l'implant comporte sa propre source d'énergie pour le fonctionnement de l'appareillage électrique (moteur ou autre) ; ceci, bien sûr, n'est pas pratique pour les implants nécessitant beaucoup d'énergie, en terme de puissance ou de durée, car il faut alors régulièrement changer la source d'énergie lors d'une opération chirurgicale.

La présente invention entend remédier aux inconvénients de l'art antérieur en proposant un dispositif de commande, selon lesquels le dispositif de mise en oeuvre est susceptible à la fois de communiquer avec le dispositif de constriction gastrique en lui envoyant des informations sous forme de données binaires et à la fois de fournir une alimentation en énergie dudit dispositif pour le fonctionnement de l'appareillage électrique qu'il comporte, à l'aide d'un moyen unique, une source d'induction. La présente invention entend également proposer un ensemble composé d'un dispositif de commande et d'un dispositif de constriction gastrique implantable selon lequel l'implant est susceptible à la fois de communiquer avec le dispositif de commande en lui envoyant des informations sous forme de données binaires et à la fois de recevoir une énergie nécessaire au fonctionnement de l'appareillage électrique qu'il comporte.

Pour ce faire, l'invention est définie dans la revendication principale.

Selon l'invention, on procède à des interruptions dans la réalisation du champ électromagnétique par ladite source d'induction afin de transmettre des informations sous forme de données binaires audit implant.

Avantageusement, ladite transmission d'information est opérée à l'aide de bits, éventuellement temporisés.

Avantageusement, ladite transmission d'information est opérée du dispositif de mise en oeuvre vers l'implant et inversement.

Avantageusement, lesdites informations concernent alternativement ou cumulativement :
- le numéro de série de l'implant ;
- l'identifiant du patient ;
- la date de première mise en service ;
- la date de la dernière intervention ;
- le nombre d'interventions ;
- l'état de l'implant ;
- l'historique du contenu des interventions ;
- les accusés de réception des transmissions ;
- la confirmation de la réalisation de l'action.

Avantageusement, les accusés de réception des transmissions sont constitués par des bits particuliers.

La présente invention se rapporte ainsi à un dispositif de commande comme défini dans la revendication 1.

Avantageusement, lesdits moyens pour permettre de procéder à des interruptions dans la réalisation du champ électromagnétique sont constitués par un onduleur couplé à une antenne émettrice. Ladite antenne est également de préférence couplée à un récepteur et ledit dispositif de mise en oeuvre comporte un module de détection et de traitement des modifications subies dans ledit champ électromagnétique.

Avantageusement, le dispositif de commande comporte un microcontrôleur relié à un dispositif d'affichage et de commande et/ou susceptible de communiquer avec un ordinateur servant d'interface.

Avantageusement, le dispositif de commande comporte également un abaisseur de tension à découpage, afin de permettre d'optimiser l'amplitude du champ électromagnétique.

La présente invention se rapporte à un ensemble comprenant un dispositif de constriction gastrique, susceptible d'être mis en oeuvre à distance et sans lien matériel par un dispositif de commande selon l'invention.

Ledit implant comporte des moyens pour permettre de procéder à des modifications dans ledit champ électromagnétique afin de transmettre des informations sous forme de données binaires audit dispositif de commande.

Avantageusement, lesdits moyens pour permettre de procéder à des modifications dans ledit champ électromagnétique sont constitués par un module de communication.

Avantageusement, lesdites informations transmises par l'implant concernent alternativement ou cumulativement :
- le numéro de série de l'implant ;
- l'identifiant du patient ;
- la date de première mise en service ;
- la date de la dernière intervention ;
- le nombre d'interventions ;
- l'historique du contenu des interventions ;
- les accusés de réception des transmissions ;
- la confirmation de la réalisation de l'action.

En outre, les accusés de réception des transmissions sont de préférence constitués par des bits particuliers.

Avantageusement, l'implant comporte un microcontrôleur relié audits moyens permettant de procéder à des modifications dans ledit champ électromagnétique.

Avantageusement, ledit appareillage électrique est un moteur sans balai à capteur(s) de position du rotor.

On comprendra mieux l'invention à l'aide de la description, faite ci-après à titre purement explicatif, d'un mode de réalisation de l'invention, en référence aux figures annexées :
- la figure 1 illustre un schéma de principe du dispositif de mise en oeuvre à distance et sans lien matériel d'un implant et de l'implant mis en oeuvre par ce dispositif, selon l'invention ; et
- la figure 2 illustre un organigramme simplifié du fonctionnement de l'ensemble dispositif de mise en oeuvre / implant.

Le dispositif de commande à distance et sans lien matériel selon l'invention, est un dispositif de commande d'un dispositif de constriction gastrique implantable (20) comportant un appareillage électrique (21), à l'aide d'un dispositif de mise en oeuvre (10), ledit dispositif de mise en oeuvre (10) comportant une source d'induction (11) constituant une source d'énergie pour le fonctionnement dudit appareillage électrique (21).

Le dispositif selon l'invention est caractérisé en ce qu'on procède à des interruptions dans la réalisation du champ électromagnétique par ladite source d'induction (11) afin de transmettre des informations sous forme de données binaires audit implant (20).

La mise en oeuvre de ce dispositif de commande est opérée une fois que l'implant a été implanté dans le corps. Elle est donc opérée par des personnes qualifiées, mais en dehors de toute intervention chirurgicale.

La transmission d'information est opérée à l'aide de bits, éventuellement temporisés, lesdits bits pouvant former des mots, comme par exemple des octets.

Le dispositif de commande (10) comporte ainsi des moyens pour permettre de procéder à des interruptions dans la réalisation du champ électromagnétique par ladite source d'induction (11) afin de transmettre des informations sous forme de données binaires audit implant (20).

Le dispositif de commande (10) selon l'invention a été mis au point pour la commande d'un implant (20) réalisant un anneau de constriction gastrique.

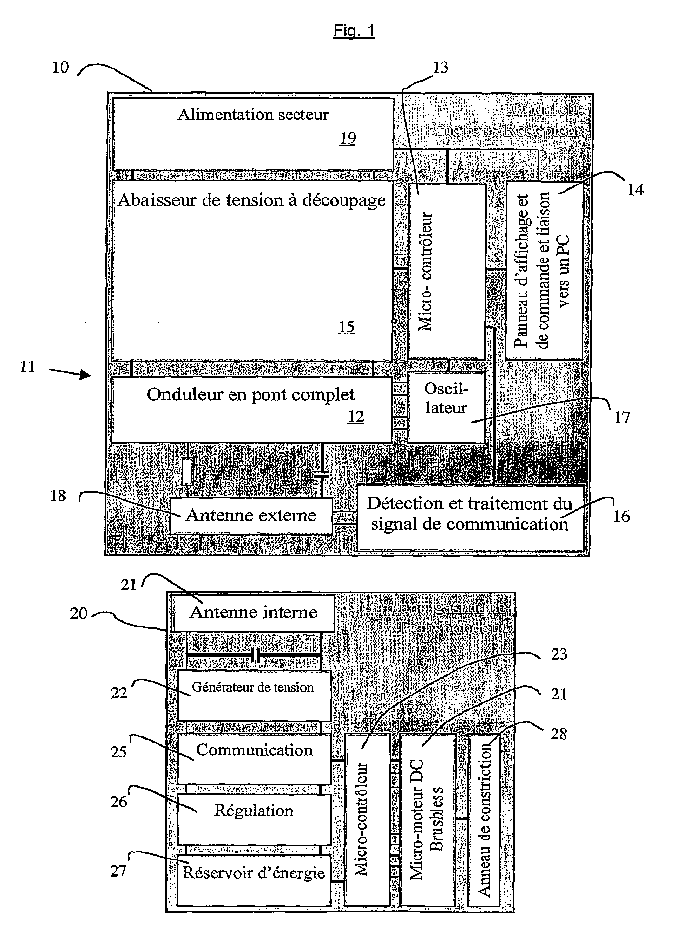

L'invention illustrée figure 1, repose sur deux éléments : d'une part un onduleur émetteur/récepteur et d'autre part un implant transpondeur.
- Onduleur émetteur/Récepteur : c'est ce système qui fournie l'énergie à l'implant gastrique, gère la communication, réalise l'interface homme/machine. Il peut être matériellement constitué d'une ou de plusieurs parties. Ce système comprend les éléments suivants :
   o L'alimentation secteur (19) : elle garantie l'isolation du secteur, intègre un correcteur de facteur de puissance (PFC), fournie la puissance nécessaire au fonctionnement de l'abaisseur (15) et de l'onduleur (12) ainsi que les tensions de commandes ;
   o Abaisseur de tension à découpage (15) : il permet de moduler l'intensité du champ électromagnétique en fonction du besoin ;
   o Onduleur en pont complet (12) : cette structure est nécessaire afin de maîtriser la tension appliquée sur le circuit résonnant. Il fournit une tension de forme carrée. La modulation de l'amplitude est uniquement gérée par l'abaisseur (15). Ceci permet de limiter la fréquence de commutation des interrupteurs électroniques.
   o Antenne externe (18) : c'est la bobine qui génère le champ électromagnétique nécessaire au fonctionnement de l'implant. C'est elle également qui reçoit les signaux provenant de l'implant (20). Elle est associée à un condensateur et une résistance pour constituer le circuit résonnant.
   o Module (16) de détection et traitement du signal de communication : ce module permet d'extraire les données en partant de la faible variation de tension aux bornes de l'antenne externe et/ou du déphasage du courant.
   o Oscillateur (17) : il génère les signaux de commande de l'onduleur en pont complet (12). Ces signaux sont calés sur une horloge très précise.
   o Microcontrôleur (13) : il pilote tous les systèmes. Il gère en particulier la communication, l'amplitude du champ électromagnétique et l'interface homme/machine.
   o Dispositif (14) d'affichage et de commande et/ou de liaison vers un ordinateur personnel : sur l'affichage se trouvent toutes les données communiquées par l'implant. Les informations, telles que le nom du patient, sont saisies grâce à un clavier intégré du type de ceux utilisés pour les ordinateurs personnels. Une liaison série permet de communiquer avec un ordinateur personnel, par exemple pour a maintenance du dispositif.
- Implant (20) transpondeur :
   o Antenne interne (24) : c'est grâce à cette bobine que l'implant est alimenté et communique avec l'extérieur. Elle est associée à un condensateur afin de réaliser un circuit résonnant calibré à la même fréquence que celle de l'onduleur.
   o Générateur de tension (22) : génère une tension à partir de l'énergie reçue par l'antenne (24) ;
   o Module (25) de communication : la communication repose sur le principe du transpondeur. La modification de la tension aux bornes de l'antenne interne entraîne un faible changement de l'amplitude de la tension aux bornes de l'antenne externe et également une modification du déphasage du courant dans le circuit résonnant externe. Pour transmettre des données, l'onduleur coupe l'induction pendant un court instant. Cette coupure est détectée par le microcontrôleur (23) de l'implant (20).
   o Module (26) de régulation : il limite la tension aux bornes du microcontrôleur (23).
   o Le réservoir d'énergie (27) : il permet au microcontrôleur (23) d'être alimenté pendant certaines phases de la communication.
   o Microcontrôleur (23) : c'est le seul circuit complexe de l'implant. Il gère toute la communication et il pilote aussi directement l'appareillage électrique (21) tout en contrôlant l'amplitude de la constriction.
   o Appareillage électrique (21) : par exemple un micro-moteur de type « DC Brushless », sans balai, muni de capteur(s) de position du rotor. Ce type de moteur, quasiment sans usure, permet par construction de connaître précisément le nombre de rotation qu'il effectue.
   o Anneau de constriction (28) : il est actionné par le micro-moteur grâce à un système vis-écrou.

L'implant (20) est alimenté par induction. L'induction est à fréquence fixe, par exemple 115,2 kHz. Toutes les formes d'ondes sont sinusoïdales.

L'implant (20) comporte un microcontrôleur (23) qui d'une part pilote l'appareillage électrique (21) constitué par le micro-moteur et d'autre part permet la communication avec le dispositif de mise en oeuvre (10) et le stockage des données.

L'implant (20) communique avec l'extérieur en modifiant la tension aux bornes de son antenne (24). Cette modification se traduit par une modification de la tension appliquée sur l'antenne externe (18) et sur le déphasage du courant. La durée de ces modifications est traitée puis le signal est interprété par le microcontrôleur (13) de l'onduleur.

Afin de communiquer de l'extérieur vers l'implant (20), on procède à des coupures du champ électromagnétique. L'implant est alors alimenté par son réservoir d'énergie (27) et mesure la durée des coupures afin d'en extraire les données.

De l'intérieur vers l'extérieur, les durées des modifications sont, par exemple, de 500µs pour un 0 logique, 1ms pour un 1 logique et 2ms pour l'accusé de réception. Un bit est transmis toutes les 2ms.

De l'extérieur vers l'intérieur, les durées des modifications sont, par exemple, de 750µs pour un 0 logique, 1,5ms pour un 1 logique et 3ms pour l'accusé de réception. Un bit transmis toutes les 3ms.

Le format des données transmises par l'implant (20) est, par exemple, le suivant :
o Numéro de série de l'implant codé sur 4 octets
o Identifiant du patient codé sur 30 octets
o Date de première mise en service sur 6 octets
o Date de la dernière intervention sur 6 octets
o Historique des positions sur 4 octets soit 8 positions (Position de l'anneau codé sur 3 bits deux positions par octet)
o Nombre d'interventions sur 1 octet

Le format des données transmises à l'implant (20) est le suivant :
o En mode programmation
   ■ Code confidentiel codé sur 1 octet
   ■ Identifiant de l'implant codé sur 4 octets
   ■ Identifiant du patient codé sur 30 octets
   ■ Date de première mise en service sur 6 octets
o En mode normal :
   ■ Identifiant de l'implant codé sur 4 octets
   ■ Nouvelle position à atteindre codée sur 1 octet
   ■ Uniquement après déplacement confirmé, date sur 6 octets

Afin de sécuriser le processus, en particulier en cas de mauvaise communication de l'implant, il est possible d'envoyer un numéro de série « passe-partout » qui permet de commander l'anneau.

Dans les deux sens de communication, chaque octet de donnée transmis est directement précédé de son adresse sur 1 octet et directement suivi par 4 bits correspondant à la somme de contrôle du mot transmis. S'il n'y a pas d'erreur, le récepteur transmet l'accusé de réception. L'émetteur envoie alors la donnée suivante. Si l'émetteur ne reçoit pas l'accusé de réception, il tente à nouveau d'envoyer la même donnée et peut opérer jusqu'à dix tentatives.

Ce type de communication totalement original permet de se prémunire de toute mauvaise interprétation ou « piratage » provenant d'un autre système à induction de même fréquence.

L'amplitude du champ électromagnétique est modulée en permanence en fonction des besoins de l'implant afin de limiter la puissance au strict nécessaire.

L'antenne interne (24) est disposée dans l'implant (20). L'antenne est disposée de manière à ce que son axe soit le plus perpendiculaire possible à la peau.

Pour la bonne compréhension de l'invention, il est important de noter que par « lien physique », on entend un lien matériel, tel qu'un câble, par exemple.

Ainsi, aucune liaison électrique par câble n'est à réaliser entre l'implant (20) et le dispositif de mise en oeuvre (10).

Afin de faire fonctionner l'implant, on applique l'antenne externe (18) sur la peau du patient. À la mise sous tension, l'amplitude du champ est optimisée pour l'obtention de la communication avec l'implant. Après diverses opérations de contrôle, l'implant transmet toutes les données. Une fois ces données reçues correctement, elles sont affichées sur le dispositif (14) d'affichage et de commande du dispositif de mise en oeuvre (10). L'opérateur peut alors choisir la nouvelle position à atteindre. Après confirmation, ces données sont envoyées à l'implant (20). Ensuite le champ augmente afin de transmettre la puissance nécessaire au micro-moteur. À la fin du déplacement, une information est envoyée au dispositif de mise en oeuvre (10), le champ revient à sa valeur nominale. Le dispositif de mise en oeuvre (10) demande à l'implant si le déplacement à été correctement effectué et en avise l'opérateur.

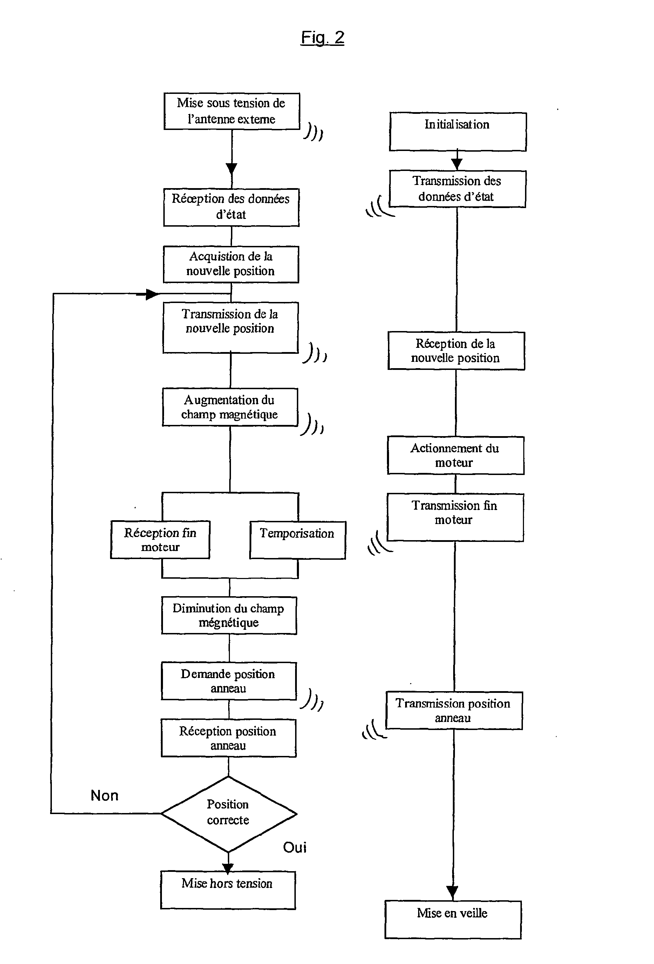

La figure 2 illustre un synoptique du système.

L'invention est décrite dans ce qui précède à titre d'exemple. Il est entendu que l'homme du métier est à même de réaliser différentes variantes de l'invention qui est définie par les revendications du brevet.

## Revendications

1. Dispositif de commande (10) sans lien matériel d'un dispositif de constriction gastrique (20) comportant un appareillage électrique (21), ledit dispositif de commande (10) comportant une source d'induction (11) constituant une source d'énergie pour le fonctionnement dudit appareillage électrique (21), **caractérisé en ce que** le dispositif de commande comporte des moyens pour permettre de procéder à des interruptions dans la réalisation du champ électromagnétique par ladite source d'induction (11) afin de transmettre des informations sous forme de données binaires audit dispositif de constriction gastrique (20).

2. Dispositif de commande (10) selon la revendication 1, **caractérisé en ce que** lesdits moyens pour permettre de procéder à des interruptions dans la réalisation du champ électromagnétique sont constitués par un onduleur (12) couplé à une antenne (24) émettrice.

3. Dispositif de commande (10) selon la revendication 2, **caractérisé en ce que** ladite antenne (24) est couplée à un récepteur.

4. Dispositif de commande (10) selon l'une quelconque des revendications 1 à 3, **caractérisé en ce qu'**il comporte un microcontrôleur (13) relié à un dispositif (14) d'affichage et de commande et/ou susceptible de communiquer avec un ordinateur servant d'interface.

5. Dispositif de commande (10) selon l'une quelconque des revendications 1 à 4, **caractérisé en ce qu'**il comporte un abaisseur de tension à découpage (15), afin de permettre d'optimiser l'amplitude du champ électromagnétique.

6. Dispositif de commande (10) selon l'une quelconque des revendications 1 à 5, **caractérisé en ce qu'**il comporte un module (16) de détection et de traitement de modifications subies dans ledit champ électromagnétique.

7. Utilisation d'un dispositif comportant une source d'induction (11) et des moyens pour permettre de procéder à des interruptions dans la réalisation du champ électromagnétique par ladite source d'induction (11), en tant que commande d'un dispositif de constriction gastrique (20) comportant un appareillage électrique (21), afin de transmettre des informations sous forme de données binaires audit dispositif de constriction gastrique (20).

8. Utilisation d'un dispositif selon la revendication 7, **caractérisé en ce que** ladite transmission d'information est opérée à l'aide de bits, éventuellement temporisés.

9. Utilisation d'un dispositif selon la revendication 7 ou 8, **caractérisé en ce que** ladite transmission d'information est opérée du dispositif de mise en oeuvre (10) vers le dispositif de constriction gastrique (20) et inversement.

10. Utilisation d'un dispositif selon la revendication 7 à 9, **caractérisé en ce que** lesdites informations concernent alternativement ou cumulativement :
- le numéro de série de l'implant ;
- l'identifiant du patient ;
- la date de la dernière intervention ;
- le nombre d'intervention ;
- l'état de l'implant ;
- historique du contenu des interventions ;
- les accusé de réception des transmissions ;
- la confirmation de la réalisation de l'action.

11. Utilisation d'un dispositif selon l'une des revendications 7 à 10, **caractérisé en ce que** les accusés de réception des transmissions sont constitués par des bits particuliers.

12. Ensemble composé d'un dispositif de commande selon l'une des revendications 1 à 6 et d'un dispositif de constriction gastrique (20) comportant un appareillage électrique (21) **caractérisé en ce que** ledit dispositif de constriction gastrique est susceptible d'être commandé à distance et sans lien matériel par ledit dispositif de commande.

13. Ensemble selon la revendication 12, **caractérisé en ce que** le dispositif de constrictions gastrique comporte des moyens pour permettre de procéder à des modifications dans ledit champ électromagnétique afin de transmettre des informations sous forme de données binaires audit dispositif de mise en oeuvre (10).

14. Ensemble selon la revendication 12 ou 13, **caractérisé en ce que** lesdits moyens pour permettre de procéder à des modifications dans ledit champ électromagnétique sont constitués par un module (25) de communication.

15. Ensemble selon l'une des revendications 12 à 14, **caractérisé en ce que** lesdites informations concernent alternativement ou cumulativement :
- le numéro de série de l'implant ;
- l'identifiant du patient ;
- la date de première mise en service ;
- la date de la dernière intervention ;
- le nombre d'interventions ;
- l'état de l'implant ;
- l'historique du contenu des interventions ;
- les accusés de réception des transmissions ;
- la confirmation de la réalisation de l'action.

16. Ensemble selon la revendication 15, **caractérisé en ce que** les accusés de réception des transmissions sont constitués par des bits particuliers.

17. Ensemble selon l'une des revendications 12 à 16, **caractérisé en ce que** ledit dispositif de constriction gastrique (20) comporte un microcontrôleur (23) relié audits moyens permettant de procéder à des modifications dans ledit champ électromagnétique.

18. Ensemble selon l'une des revendications 12 à 17, **caractérisé en ce que** ledit appareillage électrique (21) est un moteur sans balai à capteur(s) de position du rotor.

## Claims

1. Device (10) for controlling, without a hardware link, a gastric constriction device (20) comprising an electrical appliance (21), the said control device (10) comprising an induction source (11) constituting a power source for the functioning of the said electrical appliance (21), **characterised in that** the control device comprises means for proceeding with interruptions in the production of the electromagnetic field by the said induction source (11) in order to transmit information in the form of binary data to the said gastric constriction device (20).

2. A control device (10) according to claim 1, **characterised in that** the said means for making it possible to proceed with interruptions in the production of the electromagnetic field consist of an inverter (12) coupled to a transmitting antenna (24).

3. A control device (10) according to claim 2, **characterised in that** the said antenna (24) is coupled to a receiver.

4. A control device (10) according to any one of claims 1 to 3, **characterised in that** it comprises a microcontroller (13) connected to a display and control device (14) and/or able to communicate with a computer serving as an interface.

5. A control device (10) according to any one of claims 1 to 4, **characterised in that** it comprises a voltage reducer with chopping (15), to make it possible to optimise the amplitude of the electromagnetic field.

6. A control device (10) according to any one of claims 1 to 5, **characterised in that** it comprises a module (16) for detecting and processing modifications undergone in the said electromagnetic field.

7. Use of a device comprising an induction source (11) and means for proceeding with interruptions in the production of the electromagnetic field by the said induction source (11), as a control for a gastric constriction device (20) comprising an electrical appliance (21), in order to transmit information to the said gastric constriction device (20) in the form of binary data.

8. Use of a device according to claim 7, **characterised in that** the said information transmission is carried out by means of bits, possibly timed.

9. Use of a device according to claim 7 or 8, **characterised in that** the said information transmission is carried out from the implementation device (10) to the gastric constriction device (20) and vice versa.

10. Use of a device according to claims 7 to 9, **characterised in that** the said information concerned, in alternation or cumulatively:
- the serial number of the implant;
- the patient identifier;
- the date of the last intervention;
- the number of interventions;
- the state of the implant;
- history of the content of the intervention;
- acknowledgements of transmissions;
- confirmation of the performance of the action.

11. Use of a device according to one of claims 7 to 10, **characterised in that** the acknowledgements of the transmissions consist of particular bits.

12. An assembly composed of a control device according to one of claims 1 to 6 and a gastric constriction device (20) comprising an electrical appliance (21), **characterised in that** the said gastric constriction device is able to be controlled remotely and without any hardware link by the said control device.

13. An assembly according to claim 12, **characterised in that** the gastric constriction device comprises means for making it possible to proceed with modifications in the said electromagnetic field in order to transmit information to the said implementation device (10) in the form of binary data.

14. An assembly according to claim 12 or 13, **characterised in that** the said means for making it possible to proceed with modifications in the said electromagnetic field consist of a communication module (25).

15. An assembly according to one of claims 12 to 14, **characterised in that** the said information concern, in alternation or cumulatively:
- the serial number of the implant;
- the patient identifier;
- the date of first commissioning;
- the date of the last intervention;
- the number of interventions;
- the state of the implant;
- history of the content of the intervention;
- acknowledgements of the transmissions;
- confirmation of the performance of the action.

16. An assembly according to claim 15, **characterised in that** the acknowledgements of the transmissions consist of particular bits.

17. An assembly according to one of claims 12 to 16, **characterised in that** the said gastric constriction device (20) comprises a microcontroller (23) connected to the said means for proceeding with modifications in the said electromagnetic field.

18. An assembly according to one of claims 12 to 17, **characterised in that** the said electrical appliance (21) is a brushless motor with rotor position sensor or sensors.

## Patentansprüche

1. Steuervorrichtung (10) ohne materielle Verbindung für eine Mageneinschürungsvorrichtung (20) mit einem elektrischen Gerät (21), wobei die besagte Steuervorrichtung (10) eine Induktionsquelle (11) umfasst, die eine Energiequelle für den Betrieb des besagten elektrischen Geräts (21) bildet, **dadurch gekennzeichnet, dass** die Steuervorrichtung Mittel umfasst, um Unterbrechungen bei der Herstellung des elektromagnetischen Felds durch die besagte Induktionsquelle (11) zu ermöglichen, um Informationen in Form von binären Daten an die besagte Mageneinschnürungsvorrichtung (20) zu übertragen.

2. Steuervorrichtung (10) nach Anspruch 1, **dadurch gekennzeichnet, dass** die besagten Mittel zur Unterbrechung der Herstellung des elektromagnetischen Felds aus einem mit einer Sendeantenne (24) verbundenen Wechselrichter (12) bestehen.

3. Steuervorrichtung (10) nach Anspruch 2, **dadurch gekennzeichnet, dass** die besagte Antenne (24) mit einem Empfänger verbunden ist.

4. Steuervorrichtung (10) nach einem der Ansprüche 1 bis 3, **dadurch gekennzeichnet, dass** sie einem Mikrocontroller (13) umfasst, der an eine Anzeige- und Steuervorrichtung (14) angeschlossen ist und/oder mit einem als Schnittstelle dienenden Computer kommunizieren kann.

5. Steuervorrichtung (10) nach einem der Ansprüche 1 bis 4, **dadurch gekennzeichnet, dass** sie einen schneidenden Abwärtstransformator (15) umfasst, um die Optimierung der Amplitude des elektromagnetischen Felds zu ermöglichen.

6. Steuervorrichtung (10) nach einem der Ansprüche 1 bis 5, **dadurch gekennzeichnet, dass** sie ein Erfassungs- und Verarbeitungsmodul (16) von in dem besagten elektromagnetischen Feld auftretenden Änderungen umfasst.

7. Verwendung einer Vorrichtung mit einer Induktionsquelle (11) und Mitteln zur Unterbrechung der Herstellung des elektromagnetischen Felds durch die besagte Induktionsquelle (11) als Steuerung einer Mageneinschnürungsvorrichtung (20) mit einem elektrischen Gerät (21), um Informationen in Form von binären Daten an die besagte Mageneinschnürungsvorrichtung (20) zu übertragen.

8. Verwendung einer Vorrichtung nach Anspruch 7, **dadurch gekennzeichnet, dass** die besagte Informationsübertragung mit Hilfe von eventuell verzögerten Bits erfolgt.

9. Verwendung einer Vorrichtung nach Anspruch 7 oder 8, **dadurch gekennzeichnet, dass** die besagte Informationsübertragung von der Steuervorrichtung (10) zur Mageneinschnürungsvorrichtung (20) und umgekehrt erfolgt.

10. Verwendung einer Vorrichtung nach Anspruch 7 bis 9, **dadurch gekennzeichnet, dass** die besagten Informationen alternativ oder kumulativ betreffen:
- die Seriennummer des Implantats;
- die Identifikationsnummer des Patienten;
- das Datum des letzten Eingriffs;
- die Anzahl der Eingriffe;
- den Zustand des Implantats;
- die Historie des Inhalts der Eingriffe;
- die Empfangsbestätigungen der Übertragungen;
- die Bestätigung der Durchführung der Aktion.

11. Verwendung einer Vorrichtung nach einem der Ansprüche 7 bis 10, **dadurch gekennzeichnet, dass** die Empfangsbestätigungen der Übertragungen aus besonderen Bits bestehen.

12. Einheit bestehend aus einer Steuervorrichtung nach einem der Ansprüche 1 bis 6 und einer Mageneinschnürungsvorrichtung (20) mit einem elektrischen Gerät (21), **dadurch gekennzeichnet, dass** die besagte Mageneinschnürungsvorrichtung aus Entfernung und ohne materielle Verbindung mit der besagten Steuervorrichtung gesteuert werden kann.

13. Einheit nach Anspruch 12, **dadurch gekennzeichnet, dass** die Mageneinschnürungsvorrichtung Mittel umfasst, um Änderungen in dem besagten elektromagnetischen Feld vornehmen zu können, um Informationen in Form von binären Daten an die besagte Steuervorrichtung (10) zu übertragen.

14. Einheit nach Anspruch 12 oder 13, **dadurch gekennzeichnet, dass** die besagten Mittel für die Durchführung von Änderungen in dem besagten elektromagnetischen Feld aus einem Kommunikationsmodul (25) bestehen.

15. Einheit nach einem der Ansprüche 12 bis 14, **dadurch gekennzeichnet, dass** die besagten Informationen alternativ oder kumulativ betreffen:
- die Seriennummer des Implantats;
- die Identifikationsnummer des Patienten;
- das Datum der ersten Inbetriebnahme
- das Datum des letzten Eingriffs;
- die Anzahl der Eingriffe;
- den Zustand des Implantats;
- die Historie des Inhalts der Eingriffe;
- die Empfangsbestätigungen der Übertragungen;
- die Bestätigung der Durchführung der Aktion.

16. Einheit nach Anspruch 15, **dadurch gekennzeichnet, dass** die Empfangsbestätigungen der Übertragungen aus besonderen Bits bestehen.

17. Einheit nach einem der Ansprüche 12 bis 16, **dadurch gekennzeichnet, dass** die besagte Mageneinschnürungsvorrichtung (20) einen Mikrocontroller (23) umfasst, der an die Mittel für die Durchführung von Änderungen in dem besagten elektromagnetischen Feld angeschlossen ist.

18. Einheit nach einem der Ansprüche 12 bis 17, **dadurch gekennzeichnet, dass** das besagte elektrische Gerät (21) ein bürstenloser Motor mit Positionssensor(en) des Rotors ist.
